# EUROPÄISCHE PATENTSCHRIFT

(11) **EP 3 616 621 B1**
(45) Veröffentlichungstag und Bekanntmachung des Hinweises auf die Patenterteilung: **15.03.2023**
(21) Anmeldenummer: 18191913.5
(22) Anmeldetag: 31.08.2018
(51) Int. Cl.: A61B 6/00, A61B 6/03

(54) **VERFAHREN ZUR 3D DSA UND VORRICHTUNG**
METHOD AND APPARATUS FOR 3D DSA
PROCÉDÉ D'ASN TRIDIMENSIONNELLE ET DISPOSITIF

(43) Veröffentlichungstag der Anmeldung: 04.03.2020
(73) Patentinhaber: Siemens Healthcare GmbH, 91052 Erlangen (DE)
(72) Erfinder: KOWARSCHIK, Markus, 90408 Nürnberg (DE); MANHART, Michael, 90765 Fürth (DE)

(56) Entgegenhaltungen:
- US-A- 5 839 440
- MARTIN G. WAGNER ET AL: "Feasibility of intra-acquisition motion correction for 4D DSA reconstruction for applications in the thorax and abdomen", MEDICAL IMAGING 2018: IMAGE PROCESSING, 2. März 2018 (2018-03-02), Seite 40, XP055561000, DOI: 10.1117/12.2293812 ISBN: 978-1-5106-1638-7
- Robert Frysch ET AL: "Rigid Motion Compensation in Interventional C-arm CT Using Consistency Measure on Projection Data" In: "Serious Games", 1. Januar 2015 (2015-01-01), Springer International Publishing, Cham 032682, XP055559751, ISSN: 0302-9743 ISBN: 978-3-642-37803-4 Bd. 9349, Seiten 298-306, DOI: 10.1007/978-3-319-24553-9_37, * Zusammenfassung * * Sektion "1 Introduction" * * Sektion "2 Methods" *

## Beschreibung

Die Erfindung betrifft ein Verfahren zur Erstellung einer hochauflösenden dreidimensionalen Digitalen Subtraktionsangiographie eines Untersuchungsobjekts gemäß dem Patentanspruch 1 sowie eine Vorrichtung zur Durchführung eines derartigen Verfahrens gemäß dem Patentanspruch 6.

Die "einfache" digitale Subtraktionsangiographie (DSA) dient der Untersuchung von Blutgefäßen. Hierbei werden von einem zu untersuchenden Körperteil, zum Beispiel dem Gehirn, mehrere zeitlich aufeinander folgende Röntgenbilder erstellt, währenddessen wird ein Kontrastmittel eingespritzt. Es resultiert ein Röntgenbild ohne Kontrastmittel, auch als Maskenbild bezeichnet, und weitere Röntgenbilder mit Kontrastmittelverteilung. Das digitale Maskenbild wird von den nachfolgenden Röntgenbildern subtrahiert. Übrig bleiben nur die Teile, die sich unterscheiden, also im Allgemeinen genau die Blutgefäße.

Die 3D Digitale Subtraktionsangiographie (3D DSA) erlaubt die hochauflösende Darstellung z.B. kontrastierter Hirngefäße als 3D Volumina. Dazu wird typischerweise ein Protokoll bestehend aus z.B. 5s DynaCT Maskenlauf, 5s Rücklauf und 5s DynaCT Fülllauf verwendet. Die zweidimensionalen Röntgenprojektionen entstammen hierbei üblicherweise einem um den Körperteil rotierenden Aufnahmeprotokoll eines C-Bogen-Röntgengeräts (DynaCT). Aufgrund der vergleichsweise langen Aufnahmezeit besteht eine erhöhte Wahrscheinlichkeit für Patientenbewegungen während als auch zwischen den DynaCTs.

Typischerweise werden die Maskenbilder von den Füllbildern abgezogen und die subtrahierten Bilder zu einem 3D Volumen rekonstruiert. Zur Bewegungskompensation wird dabei eine 2D/2D Registrierung zwischen den einzelnen Masken- und Füllbildern verwendet. Ein Problem dieses Vorgehens besteht darin, dass 3D Bewegungen in den 2D Röntgenbildern nur eingeschränkt geschätzt und kompensiert werden können.

Es ist Aufgabe der vorliegenden Erfindung, ein Verfahren zur Erstellung einer hochauflösenden dreidimensionalen Digitalen Subtraktionsangiographie eines Untersuchungsobjekts bereitzustellen, welches eine verbesserte Bewegungskompensierung ermöglicht; des Weiteren ist es Aufgabe der Erfindung, ein für die Durchführung des Verfahrens geeignetes Röntgengerät bereitzustellen.

Die Aufgabe wird erfindungsgemäß gelöst durch ein Verfahren zur Erstellung einer hochauflösenden dreidimensionalen Digitalen Subtraktionsangiographie eines Untersuchungsobjekts gemäß dem Patentanspruch 1 und von einer Vorrichtung gemäß dem Patentanspruch 6. Vorteilhafte Ausgestaltungen der Erfindung sind jeweils Gegenstand der zugehörigen Unteransprüche.

Das erfindungsgemäße Verfahren zur Erstellung einer hochauflösenden dreidimensionalen Digitalen Subtraktionsangiographie eines Untersuchungsobjekts umfasst die folgenden Schritte: Bereitstellen oder Aufnahme eines Datensatzes eines dreidimensionalen Rotationslaufes eines Aufnahmesystems um das Untersuchungsobjekt ohne Kontrastmittelgabe (Maskenlauf), Bewegungskompensation des Datensatzes des Maskenlaufes mittels eines auf den Epipolaren Konsistenzbedingungen basierenden Verfahrens, Bereitstellen oder Aufnahme eines Datensatzes eines dreidimensionalen Rotationslaufes des Aufnahmesystems um das Untersuchungsobjekt mit Kontrastmittelgabe (Fülllauf), Bewegungskompensation des Datensatzes des Fülllaufes mittels eines auf den Epipolaren Konsistenzbedingungen basierenden Verfahrens, Rekonstruktion eines ersten Volumens aus dem kompensierten Datensatz des Maskenlaufes (Maskenvolumen) und eines zweiten Volumens aus dem kompensierten Datensatz des Fülllaufes (Füllvolumen), Rigide 3D-3D Registrierung des ersten Volumens und des zweiten Volumens relativ zueinander, und Berechnung einer hochauflösenden dreidimensionalen Digitalen Subtraktionsangiographie durch Subtraktion des Maskenvolumens vom Füllvolumen. Durch das Verfahren können Patientenbewegungen sowohl während des jeweiligen Rotationslaufes also auch zwischen zwei verschiedenen Rotationsläufen (z.B. Maskenlauf und Fülllauf) besonders effektiv entfernt werden, so dass eine qualitative sehr hochwertige, fehlerminimierte 3D Digitale Subtraktionsangiographie von Gefäßen bzw. einem Gefäßbaum z.B. aus dem Gehirn oder dem Herzen eines Patienten resultiert. Durch die Anwendung einer auf den epipolaren Konsistenzbedingungen basierenden Bewegungskompensation für jeden Rotationslauf können die Bewegungen, welche während des Rotationslaufes auftreten, kompensiert werden. Anschließend werden beide Volumina einzeln rekonstruiert und rigide miteinander registriert. Hierdurch können zwischen den Rotationsläufen auftretende Bewegungen kompensiert werden. Durch die hohe Qualität der Volumenbilder können besonders gründliche Diagnosen gestellt werden, was zu einer Verbesserung der medizinischen Versorgung des Patienten beiträgt.

Die Rotationsläufe werden im Allgemeinen mit C-Bogen Röntgengeräten in Kegelstrahlgeometrie durchgeführt (Kegelstrahl-CT-Läufe bzw. DynaCT-Läufe). Während der C-Bogen um das Untersuchungsobjekt rotiert, wird eine Vielzahl von Projektionsbildern bei unterschiedlichen Rotationswinkeln aufgenommen. Bevorzugt umfasst dabei ein Rotationslauf eine Rotation von mindestens 200° (oder 180° + Öffnungswinkel).

Eine recheneffiziente Kompensation von rigider Bewegung während einzelner C-Bogen-Rotationsläufe kann durch ein auf den Epipolaren Konsistenzbedingungen (epipolar consistency conditions = ECC) basierendes Verfahren erreicht werden. Eine solche Bewegungskompensation ist z.B. aus dem Artikel "Rigid Motion Compensation in Interventional C-arm CT Using Consistency Measure on Projection Data" von R. Frysch und G. Rose, International Conference on Medical Image Computing and Computer-Assisted Intervention, Springer, Cham, 2015, pp. 298-306, bekannt. Die epipolare Geometrie wird verwendet, um Redundanzen bei Projektionsbildern zu bestimmen. Die epipolaren Konsistenzbedingungen sind besonders robuste Konsistenzparameter, welche aus dem gesamten Projektionsdatensatz Redundanzen ermitteln, basierend auf Grangeat's bekannter fundamentaler Formel für Kegelstrahl-CT.

Nach einer Ausgestaltung der Erfindung wird das Verfahren während eines Aufnahmeprotokolls mit zuerst einem Maskenlauf und anschließend zumindest einem Fülllauf durchgeführt, also sozusagen in Kombination mit der Live-Aufnahme aller Röntgenbilddaten. Zusätzlich kann zwischen Maskenlauf und Fülllauf noch ein Rücklauf integriert sein, um den C-Bogen beim Fülllauf wieder aus derselben Ausgangsposition wie beim Maskenlauf starten zu können. Beispielsweise kann ein Protokoll bestehend aus z.B. 5s DynaCT Maskenlauf, 5s Rücklauf und 5s DynaCT Fülllauf verwendet werden.

Nach einer weiteren Ausgestaltung der Erfindung erfolgt die ECC-Bewegungskompensation des Datensatzes des Maskenlaufes direkt im Anschluss an die Aufnahme des Maskenlaufs. Hierdurch kann das Verfahren besonders zeitsparend durchgeführt werden. Je nach Dauer der Bewegungskompensation erfolgt diese also zumindest teilweise während der Aufnahme des Fülllaufes, bei Vorhandensein eines Rücklaufes auch währenddessen. Sobald der bewegungskompensierte Datensatz des Maskenlaufes vorliegt, kann auch bereits eine Rekonstruktion des Maskenvolumens durchgeführt werden. Der Datensatz des Fülllaufes wird im Anschluß an seine Aufnahme bewegungskompensiert und dann zu dem Füllvolumen rekonstruiert.

Alternativ können zuvor aufgenommene und abgespeicherte Datensätze mit einem Maskenlauf und zumindest einem Fülllauf aus einer Speichereinheit oder einer Datenbibliothek abgerufen und für das Verfahren verwendet werden.

Erfindungsgemäß werden zusätzlich Redundanzen zwischen den Datensätzen unterschiedlicher Rotationsläufe verwendet, um eine Bewegungskompensation zwischen den Rotationsläufen durchzuführen. Der Hintergrund hierbei ist, dass insbesondere bei intravenösen Injektionen Inkonsistenzen durch das Kontrastmittel zwischen den Projektionsbildern bei einem Maskenlauf und den Projektionsbildern bei einem Fülllauf im Vergleich zu Inkonsistenzen durch Bewegungen des Patienten klein sind und vernachlässigt werden können. Deshalb können zusätzliche Redundanzen zwischen den Projektionsbildern der verschiedenen Rotationsläufe, z.B. zwischen dem Maskenlauf und einem Fülllauf, genutzt werden, um sowohl eine verbesserte Kompensation von In-Plane Bewegungen während einer Rotation sowie von Bewegungen zwischen den Rotationsläufen zu bewirken.

Die Erfindung umfasst außerdem ein C-Bogen-Röntgengerät aufweisend ein Aufnahmesystem mit einem C-Bogen, an welchem eine Röntgenquelle und ein Röntgendetektor gehaltert sind und welches für eine Rotationsbewegung um ein Untersuchungsobjekt und für die Aufnahme einer Vielzahl von Projektionsbildern während der Rotationsbewegung ausgebildet ist, eine Recheneinheit zur Bearbeitung von Datensätzen zur Bewegungskompensation des Datensatzes des Maskenlaufes mittels eines auf den Epipolaren Konsistenzbedingungen basierenden Verfahrens, zur Rekonstruktion des kompensierten Datensatzes, zur rigiden 3D-3D Registrierung von rekonstruierten Volumina und zur Berechnung von dreidimensionalen Digitalen Subtraktionsangiographien, eine Systemsteuerung zur Ansteuerung des Röntgengeräts und eine Anzeigeeinheit zur Anzeige der berechneten DSA.

MARTIN G. WAGNER ET AL: "Feasibility of intra-acquisition motion correction for 4D DSA reconstruction for applications in the thorax and abdomen" beschreibt eine Technik zur bewegungskompensierten 4D-DSA-Rekonstruktion, die Anwendungen im Thorax und im Abdomen ermöglicht.

Robert Frysch ET AL: "Rigid Motion Compensation in Interventional C-arm CT Using Consistency Measure on Projection Data" beschreibt einen geometrischen Optimierungsalgorithmus zur Kompensation von Patientenbewegungen.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: ein Abfolge eines erfindungsgemäßen Verfahrens zur Erstellung eines 3D DSA; und
- FIG 2: eine Ansicht eines C-Bogen-Röntgengeräts zur Durchführung eines derartigen Verfahrens.

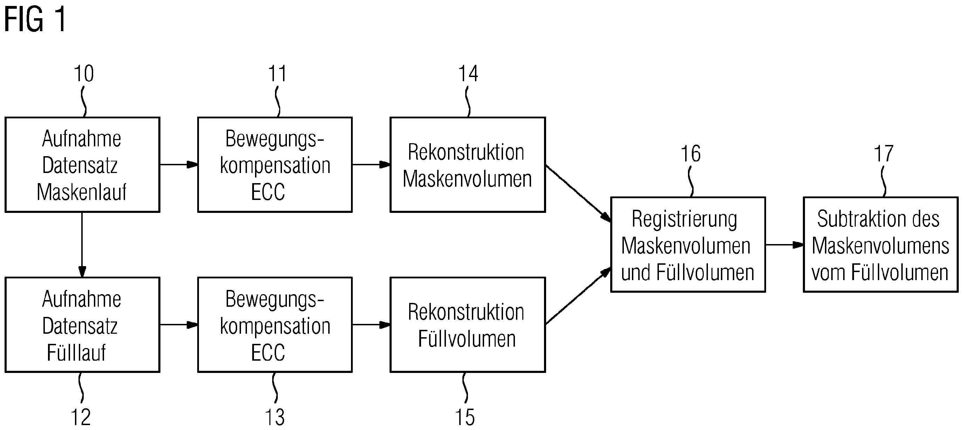

In der FIG 1 ist ein Ablauf eines erfindungsgemäßen Verfahrens zur digitalen 3D Subtraktionsangiographie gezeigt, bei welchem sowohl der Datensatz für den Maskenlauf als auch der Datensatz für den Fülllauf direkt aufgenommen werden. Alternativ können die Datensätze auch aus einer Speichereinheit oder einer Datenbibliothek abgerufen werden, nachdem sie zuvor aufgenommen und gespeichert wurden.

Die digitale 3D Subtraktionsangiographie (3D DSA) erlaubt die hochauflösende Darstellung kontrastierter Gefäßbäume, z.B. Hirngefäße oder Koronargefäße, als 3D Volumina.

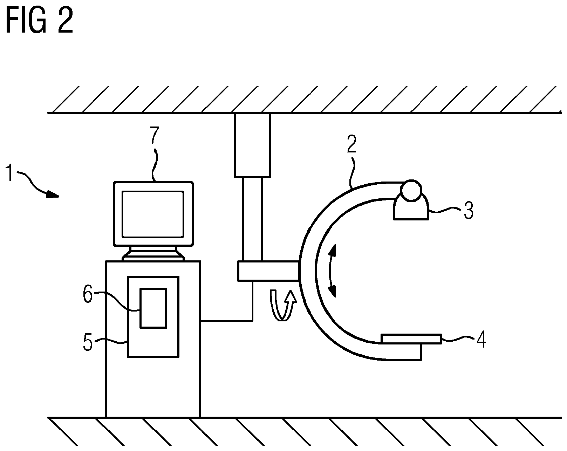

In einem ersten Schritt 10 wird mit einem C-Bogen-Röntgengerät, welches ein Aufnahmesystem mit einem C-Bogen aufweist und zur Aufnahme von dreidimensionalen Röntgenbildern ausgebildet ist, ein Datensatz während eines dreidimensionalen Rotationslaufes des Aufnahmesystems um ein Untersuchungsobjekt ohne Kontrastmittelgabe (Maskenlauf) aufgenommen. Ein beispielhaftes C-Bogen-Röntgengerät 1 ist in FIG 2 gezeigt. An dem C-Bogen 2 ist eine Röntgenquelle 3 und ein flächiger Röntgendetektor 4 angeordnet und der C-Bogen 2 ist derart ausgebildet, dass er eine Rotationsbewegung um das Untersuchungsobjekt durchführen und währenddessen eine Vielzahl von Projektionsbildern aus unterschiedlichen Projektionsrichtungen aufnehmen kann. Eine derartige Aufnahmeart mit anschließender Rekonstruktion wird z.B. als ein sogenanntes Kegelstrahl-CT oder auch DynaCT bezeichnet. Das C-Bogen-Röntgengerät wird mittels einer Steuereinheit 5 angesteuert.

Der aufgenommene Datensatz des Maskenlaufes wird in einem zweiten Schritt 11 mittels eines auf den Epipolaren Konsistenzbedingungen (epipolar consistency conditions = ECC) basierenden Verfahrens bewegungskompensiert. Durch ein derartiges Verfahren kann eine recheneffiziente Kompensation von rigider Bewegung während einzelner C-Bogen-Rotationsläufe (Kegelstrahl-CT-Läufe bzw. DynaCT-Läufe) erreicht werden. Eine solche Bewegungskompensation ist z.B. aus dem Artikel "Rigid Motion Compensation in Interventional C-arm CT Using Consistency Measure on Projection Data" von R. Frysch und G. Rose, International Conference on Medical Image Computing and Computer-Assisted Intervention, Springer, Cham, 2015, pp. 298-306, bekannt. Die epipolare Geometrie wird hierbei verwendet, um Redundanzen bei Projektionsbildern zu bestimmen. Die epipolaren Konsistenzbedingungen sind besonders robuste Konsistenzparameter, welche aus dem gesamten Projektionsdatensatz Redundanzen ermitteln, basierend auf Grangeat's bekannter fundamentaler Formel für Kegelstrahl-CT. Dazu werden mit Hilfe projektiver Geometrie redundante Linienintegrale zwischen den Projektionsbildern gefunden. Die Geometrie wird in einem iterativen Verfahren so angepasst, dass eine Fehlermetrik bezüglich dieser Redundanzen optimiert wird. Dieses rein auf Projektionsbildern basierte Verfahren ermöglicht so eine besonders recheneffiziente Schätzung starrer Bewegungsmuster.

In einem dritten Schritt 12 wird mittels desselben C-Bogen-Röntgengeräts ein Datensatz während eines dreidimensionalen Rotationslaufes des Aufnahmesystems um das Untersuchungsobjekt bei Kontrastmittelgabe (Fülllauf) aufgenommen. Der Rotationslauf als solcher unterscheidet sich bevorzugt von dem Maskenlauf nicht hinsichtlich Dauer, überstrichenem Winkel und Anzahl der Projektionsbilder. Dem Patienten wird allerdings vor der Durchführung des Fülllaufes ein Kontrastmittel in die zu untersuchenden Gefäße injiziert.

Der aufgenommene Datensatz des Fülllaufes wird in einem vierten Schritt 13 mittels desselben auf den Epipolaren Konsistenzbedingungen basierenden Verfahrens bewegungskompensiert wie der Datensatz des Maskenlaufes. Die Bewegungskompensierung der Datensätze wird beispielsweise mittels einer Berechnungseinheit 6 des C-Bogen-Röntgengeräts durchgeführt.

Typischerweise wird zur Erstellung von 3D DSA ein Aufnahmeprotokoll bestehend aus Maskenlauf, Rücklauf und Fülllauf verwendet. Auch bei dem vorliegenden Verfahren wird ein bevorzugt ein solches Aufnahmeprotokoll durchgeführt. Die Dauer jedes Laufs ist im Allgemeinen wenige Sekunden, so kann das Aufnahmeprotokoll z.B. 5s DynaCT Maskenlauf, 5s Rücklauf und 5s DynaCT Fülllauf umfassen. Bei dem verwendeten Verfahren kann die Bewegungskompensation des Maskenlaufes z.B. teilweise während des Rücklaufes und/oder des Fülllaufes durchgeführt werden. Dadurch kann das Verfahren besonders schnell durchgeführt werden.

Nachdem der Datensatz des Maskenlaufs bewegungskompensiert vorliegt, wird er in einem fünften Schritt 14 zu einem Volumen rekonstruiert (Maskenvolumen). Ebenso wird nach Vorliegen des bewegungskompensierten Datensatzes des Fülllaufes dieser zu einem Volumen rekonstruiert (Füllvolumen). Für die Rekonstruktionen werden bekannte Rekonstruktionsalgorithmen verwendet, die Rekonstruktionen können z.B. von der Berechnungseinheit 6 des C-Bogen-Röntgengeräts durchgeführt werden.

Der Ablauf des Verfahrens ist nicht zwingend vorgeschrieben. Während im Allgemeinen der Maskenlauf vor dem Fülllauf stattfinden sollte (siehe Aufnahmeprotokoll), kann die Durchführung der Bewegungskompensation des Maskenlaufs auch erst nach dem Fülllauf stattfinden, vor der Bewegungskompensation des Fülllaufes, gleichzeitig dazu oder danach. Auch die Rekonstruktionen der bewegungskompensierten Datensätze von Maskenlauf und Fülllauf können in ihrer Reihenfolge nach Bedarf variiert werden. Es kann bei Bedarf auch mehr als ein Fülllauf durchgeführt werden. In einem solchen Fall werden dann alle Füllläufe entsprechend bewegungskompensiert und zu Füllvolumina rekonstruiert.

Wenn sowohl das Maskenvolumen als auch das Füllvolumen vorliegen, wird in einem siebten Schritt 16 eine rigide Registrierung der beiden Volumina zueinander durchgeführt. Dafür kann z.B. ein Verfahren wie in dem Artikel von Paul Viola et al: Alignment by Maximization of Mutual Information; Int. Journal of Computer Vision, 24(2) pp. 137-154, 1997, verwendet werden. Durch die rigide Registrierung der Volumina zueinander wird eine Bewegung des Untersuchungsobjekts zwischen dem Maskenlauf und dem Fülllauf kompensiert.

Anschließend wird in einem achten Schritt 17 durch Subtraktion des Maskenvolumens von dem Füllvolumen eine dreidimensionale digitale Subtraktionsangiographie berechnet. Diese kann ebenfalls von der Berechnungseinheit 6 durchgeführt werden. Die 3D DSA kann anschließend an einer Anzeigeeinheit 7 des C-Bogen-Röntgengeräts angezeigt werden.

Außerdem können an geeigneter Stelle des Verfahrens zusätzliche Redundanzen zwischen den Projektionsbildern der verschiedenen Rotationsläufe, z.B. zwischen dem Maskenlauf und dem Fülllauf, genutzt werden, um eine verbesserte Kompensation von Bewegungen des Patienten sowohl während als auch zwischen den Rotationsläufen zu bewirken.

Das vorgeschlagene Verfahren erlaubt die recheneffiziente Kompensation rigider 3D Bewegung des Untersuchungsobjekts (z.B. eines Gefäßbaumes des Kopfes oder Herzens eines Patienten) sowohl während als auch zwischen den Rotationsläufen zur Aufnahme von Datensätzen für die 3D DSA.

Die Erfindung lässt sich in folgender Weise kurz zusammenfassen: Die Erfindung betrifft ein Verfahren zur Erstellung einer hochauflösenden dreidimensionalen Digitalen Subtraktionsangiographie eines Untersuchungsobjekts mit den folgenden Schritten: Bereitstellen oder Aufnahme eines Datensatzes eines dreidimensionalen Rotationslaufes eines Aufnahmesystems um das Untersuchungsobjekt ohne Kontrastmittelgabe (Maskenlauf), Bewegungskompensation des Datensatzes des Maskenlaufes mittels eines auf den Epipolaren Konsistenzbedingungen basierenden Verfahrens, Bereitstellen oder Aufnahme eines Datensatzes eines dreidimensionalen Rotationslaufes des Aufnahmesystems um das Untersuchungsobjekt mit Kontrastmittelgabe (Fülllauf), Bewegungskompensation des Datensatzes des Fülllaufes mittels eines auf den Epipolaren Konsistenzbedingungen basierenden Verfahrens, Rekonstruktion eines ersten Volumens aus dem kompensierten Datensatz des Maskenlaufes (Maskenvolumen) und eines zweiten Volumens aus dem kompensierten Datensatz des Fülllaufes (Füllvolumen), Rigide 3D-3D Registrierung des ersten Volumens und des zweiten Volumens relativ zueinander, und Berechnung einer hochauflösenden dreidimensionalen Digitalen Subtraktionsangiographie durch Subtraktion des Maskenvolumens vom Füllvolumen. Außerdem umfasst die Erfindung ein C-Bogen-Röntgengerät zur Durchführung des Verfahrens.

## Patentansprüche

1. Verfahren zur Erstellung einer hochauflösenden dreidimensionalen Digitalen Subtraktionsangiographie eines Untersuchungsobjekts mit den folgenden Schritten:
• Bereitstellen oder Aufnahme eines Datensatzes eines dreidimensionalen Rotationslaufes eines Aufnahmesystems um das Untersuchungsobjekt ohne Kontrastmittelgabe (Maskenlauf),
• Bewegungskompensation des Datensatzes des Maskenlaufes mittels eines auf den Epipolaren Konsistenzbedingungen basierenden Verfahrens,
• Bereitstellen oder Aufnahme eines Datensatzes eines dreidimensionalen Rotationslaufes des Aufnahmesystems um das Untersuchungsobjekt mit Kontrastmittelgabe (Fülllauf),
• Bewegungskompensation des Datensatzes des Fülllaufes mittels eines auf den Epipolaren Konsistenzbedingungen basierenden Verfahrens,
• Rekonstruktion eines ersten Volumens aus dem kompensierten Datensatz des Maskenlaufes (Maskenvolumen) und eines zweiten Volumens aus dem kompensierten Datensatz des Fülllaufes (Füllvolumen),
• Rigide 3D-3D Registrierung des ersten Volumens und des zweiten Volumens relativ zueinander, und
• Berechnung einer hochauflösenden dreidimensionalen Digitalen Subtraktionsangiographie durch Subtraktion des Maskenvolumens vom Füllvolumen,
wobei zusätzlich Redundanzen zwischen den Datensätzen unterschiedlicher Rotationsläufe verwendet werden, um eine Bewegungskompensation zwischen und/oder während den Rotationsläufen durchzuführen.

2. Verfahren nach Anspruch 1, welches während eines Aufnahmeprotokolls mit zuerst einem Maskenlauf und anschließend zumindest einem Fülllauf durchgeführt wird.

3. Verfahren nach Anspruch 2, wobei die Bewegungskompensation des Datensatzes des Maskenlaufes direkt im Anschluss an die Aufnahme des Maskenlaufs, insbesondere während der Aufnahme des Fülllaufes, erfolgt.

4. Verfahren nach Anspruch 1, wobei die Datensätze aus einem Datenspeicher abgerufen werden.

5. Verfahren nach einem der vorangehenden Ansprüche, wobei das auf den Epipolaren Konsistenzbedingungen basierende Verfahren zunächst mittels der Epipolaren Konsistenzbedingungen Redundanzen der Projektionsebenen in der Vielzahl von Projektionsbildern des Rotationslaufes bestimmt und diese für die Kompensation von Bewegungen des Untersuchungsobjekts während des Rotationslaufes verwendet.

6. C-Bogen Röntgengerät zur Durchführung eines Verfahrens nach einem der Ansprüche 1 bis 5, aufweisend ein Aufnahmesystem mit einem C-Bogen, an welchem eine Röntgenquelle und ein Röntgendetektor gehaltert sind und welches für eine Rotationsbewegung um ein Untersuchungsobjekt und für die Aufnahme einer Vielzahl von Projektionsbildern während der Rotationsbewegung ausgebildet ist, eine Recheneinheit zur Bearbeitung von Datensätzen zur Bewegungskompensation des Datensatzes des Maskenlaufes mittels eines auf den Epipolaren Konsistenzbedingungen basierenden Verfahrens, zur Rekonstruktion des kompensierten Datensatzes, zur rigiden 3D-3D Registrierung von rekonstruierten Volumina und zur Berechnung von dreidimensionalen Digitalen Subtraktionsangiographien, eine Systemsteuerung zur Ansteuerung des Röntgengeräts und eine Anzeigeeinheit zur Anzeige der berechneten DSA.

## Claims

1. Method for producing a high-resolution three-dimensional digital subtraction angiography image of an examination object, comprising the following steps:
• Provision or recording of a data set of a three-dimensional rotational run of an imaging system around the examination object without administration of contrast agent (mask run),
• Motion compensation of the data set of the mask run by means of a method based on the epipolar consistency conditions,
• Provision or recording of a data set of a three-dimensional rotational run of the imaging system around the examination object with administration of contrast agent (fill run),
• Motion compensation of the data set of the fill run by means of a method based on the epipolar consistency conditions,
• Reconstruction of a first volume from the compensated data set of the mask run (mask volume) and a second volume from the compensated data set of the fill run (fill volume),
• Rigid 3D-3D registration of the first volume and second volume relative to one another, and
• Calculation of a high-resolution three-dimensional digital subtraction angiography image by subtracting the mask volume from the fill volume,
wherein redundancies between the data sets for different rotational runs are additionally used to perform motion compensation between and/or during the rotational runs.

2. Method according to claim 1, which is performed during a scanning protocol initially with one mask run followed by at least one fill run.

3. Method according to claim 2, wherein the motion compensation of the data set of the mask run is performed directly after the recording of the mask run, in particular during the recording of the fill run.

4. Method according to claim 1, wherein the data sets are retrieved from a data storage unit.

5. Method according to one of the preceding claims, wherein the method based on the epipolar consistency conditions first identifies, by means of the epipolar consistency conditions, redundancies in the projection planes in the large number of projection images of the rotational run and uses these for motion compensation of the examination object during the rotational run.

6. C-arm x-ray device for performing a method according to one of claims 1 to 5, having an imaging system with a C-arm, on which an x-ray source and an x-ray detector are mounted and which is designed for a rotational movement around an examination object and for recording a large number of projection images during the rotational movement, a computing unit for processing data sets for motion compensation of the data set of the mask run by means of a method based on the epipolar consistency conditions, for the reconstruction of the compensated data set, for the rigid 3D-3D registration of reconstructed volumes, and for the calculation of three-dimensional digital subtraction angiography images, a system controller for actuating the x-ray device, and a display unit for displaying the calculated DSA.

## Revendications

1. Procédé d'établissement d'une angiographie de soustraction numérique tridimensionnelle à haute résolution d'un objet à examiner comprenant les stades suivants :
• on se procure ou on enregistre un ensemble de données d'une marche en rotation tridimensionnelle d'un système d'enregistrement autour de l'objet à examiner, sans dose d'agent de contraste (marche masquée),
• on compense en mouvement l'ensemble de données de la marche masquée au moyen d'un procédé reposant sur des conditions de consistance épipolaires,
• on se procure ou on enregistre un ensemble de données d'une marche en rotation tridimensionnelle du système d'enregistrement autour de l'objet à examiner, avec dose d'agent de contraste (marche complète),
• on compense en mouvement l'ensemble de données de la marche complète au moyen d'un procédé reposant sur les conditions de consistance épipolaires,
• on reconstruit un premier volume à partir de l'ensemble de données compensé de la marche masquée (volume masqué) et d'un deuxième volume à partir de l'ensemble de données compensé de la marche complète (volume complet),
• on enregistre en 3D-3D, de manière rigide, le premier volume et le deuxième volume l'un par rapport à l'autre, et
• on calcule une angiographie de soustraction numérique tridimensionnelle à haute résolution par soustraction du volume masqué du volume complet,
dans lequel on utilise des redondances supplémentaires entre les ensembles de données de marches en rotation différentes pour effectuer une compensation en mouvement entre et/ou pendant les marches en rotation.

2. Procédé suivant la revendication 1, que l'on effectue pendant un protocole d'enregistrement avec d'abord une marche masquée et ensuite au moins une marche complète.

3. Procédé suivant la revendication 2, dans lequel la compensation en mouvement de l'ensemble de données de la marche masquée s'effectue immédiatement à la suite de l'enregistrement de la marche masquée, notamment pendant l'enregistrement de la marche complète.

4. Procédé suivant la revendication 1, dans lequel on appelle les ensembles de données d'une mémoire de données.

5. Procédé suivant l'une des revendications précédentes, dans lequel le procédé, reposant sur les conditions de consistance épipolaires, détermine d'abord au moyen des conditions de consistance épipolaires des redondances des plans de projection dans la pluralité d'images de projection de la marche en rotation et les utilisent pour la compensation de déplacements de l'objet à examiner pendant la marche en rotation.

6. Appareil de rayons X à arceau en C pour effectuer un procédé suivant l'une des revendications 1 à 5, comportant un système d'enregistrement ayant un arceau en C, sur lequel une source de rayons X et un détecteur de rayons X sont fixés, et qui est constitué pour un mouvement en rotation autour d'un objet à examiner et pour l'enregistrement d'une pluralité d'images de projection pendant le mouvement en rotation, une unité de calcul pour le traitement d'ensemble de données pour la compensation en mouvement de l'ensembles de données de la marche masquée au moyen d'un procédé reposant sur les conditions de consistance épipolaires, pour la reconstruction de l'ensemble de données compensé, pour l'enregistrement 3D-3D de manière rigide de volumes reconstruits, et pour le calcul d'angiographies de soustraction numériques tridimensionnelles, une commande de système pour la commande de l'appareil à rayons X et une unité d'affichage pour l'affichage des ASN calculées.
